# DEMANDE DE BREVET EUROPEEN

(11) **EP 1 972 616 A1**
(43) Date de publication de la demande: **24.09.2008**
(21) Numéro de dépôt: 08012005.8
(22) Date de dépôt: 01.10.2004
(51) Int. Cl.: C07C 271/44, C07C 271/48, C07C 269/04, A61K 31/27

(54) **Dérivés d'arylalkylcarbamates, leur préparation et leur application en thérapeutique**

(30) Priorité: 03.10.2003 FR 0311615
(62) Demande divisionnaire de: 04817093.0
(71) Demandeur: Sanofi-Aventis, 75013 Paris (FR)
(72) Inventeur: Abouabdellah, Ahmed, 75013 Paris (FR); Almario, Garcia Antonio, 75013 Paris (FR)
(74) Mandataire: Gaslonde, Aude

(57) **Abrégé**

Composé répondant à la formule générale (I) : 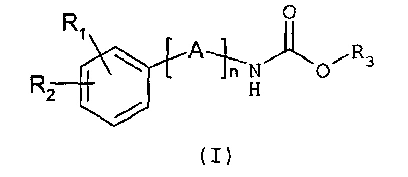 dans laquelle n représente un nombre entier compris entre 1 et 4 ; A représente un groupe C₁₋₂-alkylène ; R₁ représente un atome d'hydrogène ; R₂ représente un atome d'hydrogène, un groupe phényl ou phényloxy ; R₃ représente un groupe phényle éventuellement substitué ; à l'état de base, de sel d'addition à un acide, d'hydrate ou de solvat.

Application en thérapeutique.

## Description

L'invention a pour objet des dérivés d'arylalkylcarbamates, leur préparation et leur application en thérapeutique.

Les composés de l'invention répondent à la formule générale (I) : 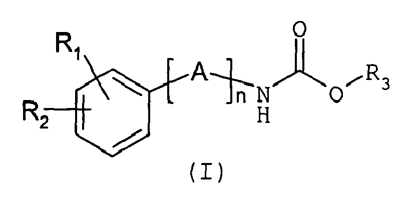 dans laquelle
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;
Z représente un groupe C₃₋₇-cycloalkyle, de formule : 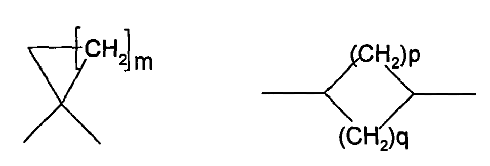 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy et
R₆ et R₇ représentent indépendamment l'un de l'autre un groupe C₁₋₃-alkyle, un phényle.

Dans le cadre de l'invention, les composés de formule générale (I) peuvent donc comporter plusieurs groupes A identiques ou différents entre eux.

Les composés suivants ne font pas partie de l'invention :
- benzylcarbamate de 2,2,2-trifluoroéthyle;
- 4-méthoxybenzylcarbamate de 2,2,2-trifluoroéthyle ;
- 2-[4-(phénylméthoxy)phényl]éthylcarbamate de 4-nitrophényle;
- 2-(4-chlorophényl)éthylcarbamate de 4-chloro-2-nitrophényle;
- 2-(3,4-diméthoxyphényl)éthylcarbamate de 4-nitrophényle;
- 2-(3,4-diméthoxyphényl)éthylcarbamate de phényle;
- 2-(4-méthylphényl)éthylcarbamate de 4-cyanophényle;
- 2-(4-chlorophényl)éthylcarbamate de 2,4,5-trichlorophényle;
- 4-chlorobenzylcarbamate de phényle;
- 4-méthoxybenzylcarbamate de phényle;
- 2-(4-méthoxyphényl)éthylcarbamate de 4-fluorophényle;
- 3-nitrobenzylcarbamate de phényle;
- 4-méthoxybenzylcarbamate de 4-cyanophényle;
- 3-chlorobenzylcarbamate de phényle;
- 3,4-dichlorobenzylcarbamate de phényle;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(3-thiényl)phényl]propylcarbamate de phényle ;
- 2-[4-(2-amino-4-thiazolyl)phényl]éthylcarbamate de phényle ;
- 4-bromobenzylcarbamate de 2,3,4,5,6-pentafluorophényle.

Parmi les composés de formule générale (I), un premier sous-groupe de composés est constitué des composés pour lesquels :
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C=C- ;

Z représente un groupe C₃₋₇-cycloalkyle, de formule : 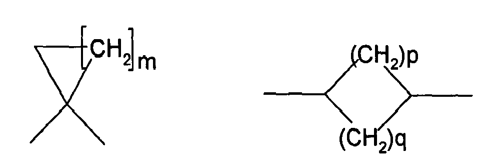 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'halogène ou un groupe nitro, hydroxyle, C₁₋₃-alkyle, C₁₋₃-alcoxy ; et
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy.

Les composés suivants ne font pas partie du premier sous-groupe de composés ci-dessus :
- 4-méthoxybenzylcarbamate de 2,2,2-trifluoroéthyle ;
- 2-(4-chlorophényl)éthylcarbamate de 4-chloro-2-nitrophényle;
- 2-(3,4-diméthoxyphényl)éthylcarbamate de 4-nitrophényle;
- 2-(3,4-diméthoxyphényl)éthylcarbamate de phényle;
- 2-(4-méthylphényl)éthylcarbamate de 4-cyanophényle;
- 2-(4-chlorophényl)éthylcarbamate de 2,4,5-trichlorophényle;
- 4-chlorobenzylcarbamate de phényle;
- 4-méthoxybenzylcarbamate de phényle;
- 2-(4-méthoxyphényl)éthylcarbamate de 4-fluorophényle;
- 3-nitrobenzylcarbamate de phényle;
- 4-méthoxybenzylcarbamate de 4-cyanophényle;
- 3-chlorobenzylcarbamate de phényle;
- 3,4-dichlorobenzylcarbamate de phényle;
- 2-(4-hydroxyphényl)éthylcarbamate de 4-nitrophényle ;
- 4-bromobenzylcarbamate de 2,3,4,5,6-pentafluorophényle.

Parmi les composés de formule générale (I), un second sous-groupe de composés est constitué des composés pour lesquels :
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 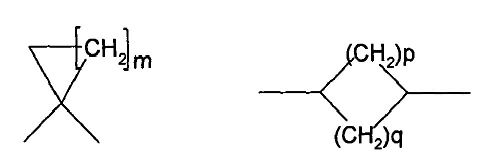 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène ou un groupe cyano, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy et
R₆ et R₇ représentent indépendamment l'un de l'autre un groupe C₁₋₃-alkyle, un phényle.

Les composés suivants ne font pas partie du second sous-groupe de composés ci-dessus :
- benzylcarbamate de 2,2,2-trifluoroéthyle;
- 2-[4-(phénylméthoxy)phényl]éthylcarbamate de 4-nitrophényle;
- 2-[4-(2-éthyl-5,7-diméthyl-3H-imidazo[4,5-b]pyridin-3-yl)phényl]éthylcarbamate de phényle ;
- 2-[4-(3-thiényl)phényl]propylcarbamate de phényle ;
- 2-[4-(2-amino-4-thiazolyl)phényl]éthylcarbamate de phényle.

Parmi les composés de formule générale (I), une première famille de composés est constituée des composés pour lesquels :
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 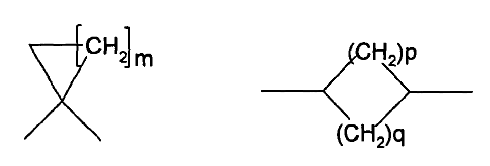 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furo pyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy et
R₆ et R₇ représentent indépendamment l'un de l'autre un groupe C₁₋₃-alkyle, un phényle ;
à la condition que
quand R₃ représente un groupe phényle,
si A représente un propylène, alors R₂ ne représente pas un thiényle.

Les composés suivants ne font pas partie de la première famille de composés définie ci-dessus :
- benzylcarbamate de 2,2,2-trifluoroéthyle ;
- 4-méthoxybenzylcarbamate de 2,2,2-trifluoroéthyle ;
- 2-[4- (phénylméthoxy) phényl]éthylcarbamate de 4-nitrophényle ;
- 2-(4-chlorophényl)éthylcarbamate de 4-chloro-2-nitrophényle ;
- 2-(3,4-diméthoxyphényl)éthylcarbamate de 4-nitrophényle ;
- 2-(3,4-diméthoxyphényl)éthylcarbamate de phényle ;
- 2-(4-méthylphényl)éthylcarbamate de 4-cyanophényle ;
- 2-(4-chlorophényl)éthylcarbamate de 2,4,5-trichlorophényle ;
- 4-chlorobenzylcarbamate de phényle ;
- 4-méthoxybenzylcarbamate de phényle ;
- 2-(4-méthoxyphényl)éthylcarbamate de 4-fluorophényle ;
- 3-nitrobenzylcarbamate de phényle ;
- 4-méthoxybenzylcarbamate de 4-cyanophényle ;
- 3-chlorobenzylcarbamate de phényle ;
- 3,4-dichlorobenzylcarbamate de phényle ;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(2-amino-4-thiazolyl)phényl]éthylcarbamate de phényle ;
- 4-bromobenzylcarbamate de 2,3,4,5,6-pentafluorophényle.

Parmi les composés de formule générale (I), une deuxième famille de composés est constituée des composés pour lesquels :
- quand R₃ représente un groupe 2,2,2-trifluoroéthyle,alors n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 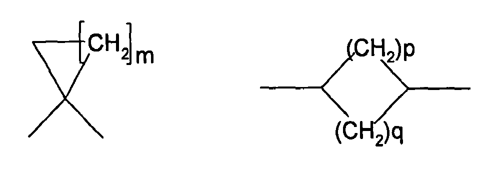 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ; et
R₆ et R₇ représentent indépendamment l'un de l'autre un groupe C₁₋₃-alkyle, un phényle ;
- quand R₃ représente un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy, alors
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 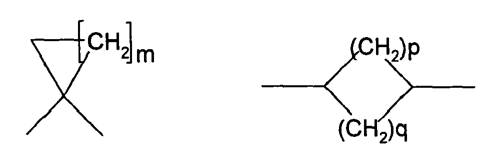 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ; et
R₆ et R₇ représentent indépendamment l'un de l'autre un groupe C₁₋₃-alkyle, un phényle.

Les composés suivants ne font pas partie de la deuxième famille de composés définie ci-dessus :
- benzylcarbamate de 2,2,2-trifluoroéthyle ;
- 4-méthoxybenzylcarbamate de 2,2,2-trifluoroéthyle ;
- 2-[4-(phénylméthoxy)phényl]éthylcarbamate de 4-nitrophényle ;
- 2-(4-chlorophényl)éthylcarbamate de 4-chloro-2-nitrophényle ;
- 2-(3,4-diméthoxyphényl)éthylcarbamate de 4-nitrophényle ;
- 2-(3,4-diméthoxyphényl)éthylcarbamate de phényle ;
- 2-(4-méthylphényl)éthylcarbamate de 4-cyanophényle ;
- 2-(4-chlorophényl)éthylcarbamate de 2,4,5-trichlorophényle ;
- 4-chlorobenzylcarbamate de phényle ;
- 4-méthoxybenzylcarbamate de phényle ;
- 2-(4-méthoxyphényl)éthylcarbamate de 4-fluorophényle ;
- 3-nitrobenzylcarbamate de phényle ;
- 4-méthoxybenzylcarbamate de 4-cyanophényle :
- 3-chlorobenzylcarbamate de phényle ;
- 3,4-dichlorobenzylcarbamate de phényle ;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(2-amino-4-thiazolyl)phényl]éthylcarbamate de phényle ;
- 4-bromobenzylcarbamate de 2,3,4,5,6-pentafluorophényle.

Parmi les composés de formule générale (I), une troisième famille de composés est constituée des composés pour lesquels :
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃-₇-cycloalkyle, de formule : 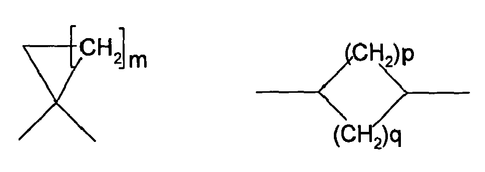 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-0-, 4-pipérazinyle éventuellement substitué par un C₁₋₃-alkyle
ou par un benzyle ;
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy et
R₆ et R₇ représentent indépendamment l'un de l'autre un groupe C₁₋₃-alkyle, un phényle ;
à la condition que
- quand R₃ représente un groupe 2,2,2-trifluoroéthyle, si A représente un méthylène et si R₁ représente un hydrogène, alors R₂ n'est ni un hydrogène, ni un méthoxy ;
   si A représente un méthylène et si R₂ représente un hydrogène, alors R₁ n'est ni un hydrogène, ni un méthoxy ;
- quand R₃ représente un groupe phényle,
   si A représente un méthylène, alors ni R₁, ni R₂ ne représentent un atome de chlore, un groupe méthoxy ou nitro ; si A représente un éthylène, alors
   ni R₁, ni R₂ ne représentent un méthoxy ;
   R₂ ne représente ni un 2-éthyl-5,7-diméthyl-3H-imidazo[4,5-b]-pyridin-3-yle ni un 2-amino-4-thiazolyle ;
   si A représente un propylène, alors R₂ ne représente pas un 3-thiényle ;
- quand R₃ représente un groupe phényle substitué par un à cinq atomes de chlore, de fluor ou groupes nitro, cyano, si A représente un méthylène, alors ni R₁, ni R₂ ne représentent un méthoxy ou un atome de brome ;
   si A représente un éthylène, alors
   ni R₁, ni R₂ ne représentent un atome de chlore, un groupe hydroxyle, méthyle ou méthoxy ;
   R₂ ne représente pas un phénylméthoxy.

Parmi les composés de formule générale (I), une quatrième famille de composés est constituée des composés pour lesquels :
- quand R₃ représente un groupe 2,2,2-trifluoroéthyle,alors n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 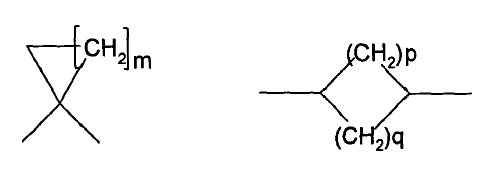 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'halogène
ou un groupe cyano, nitro, hydroxyle, C₁₋₃-alkyle, C₂₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ; et
R₆ et R₇ représentent indépendamment l'un de l'autre un groupe C₁₋₃-alkyle, un phényle ;
- quand R₃ représente un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy, alors
   n représente un nombre entier compris entre 1 et 6 ;
   A est choisi parmi un ou plusieurs groupes X, Y et/ou Z ;
   X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
   Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 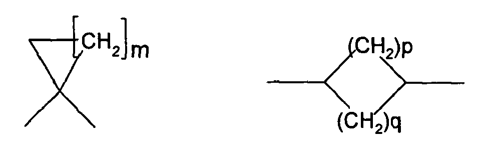 m représente un nombre entier allant de 1 à 5 ;
p et q représentent des nombres entiers et sont définis tels que p+q soit un nombre allant de 1 à 5 ;
R₁ représente un atome d'hydrogène, d'iode, ou un groupe cyano, C₂₋₃-alkyle, C₂₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'iode
ou un groupe cyano, C₂₋₃-alkyle, C₂₋₃-alcoxy, C₁₋₃-thioalkyle, C₁₋₃-fluoroalkyle, C₁₋₃-fluoroalcoxy, C₁₋₃-fluorothioalkyle, ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, furanyle, pyrrolyle, imidazolyle, pyrazolyle, oxazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -0- (C₁₋₃-alkylène) -O-, 4-pipérazinyle éventuellement substitué par un C₁₋₃-alkyle ou par un benzyle ; et
R₆ et R₇ représentent indépendamment l'un de l'autre un groupe C₁₋₃-alkyle, un phényle.

Parmi les composés de formule générale (I), une cinquième famille de composés est constituée des composés pour lesquels :
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; ou un groupe -C≡C- ;

Z représente un groupe C₃₋₇-cycloalkyle, de formule : 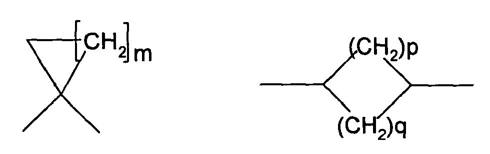 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, un groupe phényle ou phényloxy, ce groupe étant éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy et
R₆ et R₇ représentent indépendamment l'un de l'autre un groupe C₁₋₃-alkyle, un phényle ;
le benzylcarbamate de 2,2,2-trifluoroéthyle étant exclu.

Parmi les composés de formule générale (I), une sixième famille de composés est constituée des composés pour lesquels :
n représente un nombre entier compris entre 1 et 6 ;
A est un groupe X ;
les groupes A étant identiques ou différents entre eux quand n est un nombre entier compris entre 2 et 6 ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₃ représente
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy et
R₆ et R₇ représentent indépendamment l'un de l'autre un groupe C₁₋₃-alkyle, un phényle ; à la condition que :
   - lorsque R₃ représente un groupe 2,2,2-trifluoroéthyle et que le groupe -[A]ₙ- représente un groupe -CH₂-,
      alors R₁ est différent d'un atome d'hydrogène ou d'un groupe méthoxy ;
   - lorsque R₃ représente un groupe phényle éventuellement substitué et que le groupe -[A]ₙ- représente un groupe -CH₂-, -CH₂CH₂-, -CH(CH₃)-, -CH(CH₃)-CH₂-,
      alors R₁ est différent d'un atome d'hydrogène ou de chlore ou d'un groupe méthyle ou méthoxy et R₂ est différent d'un atome d'hydrogène.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases
ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 12, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₃-alkyle représente une chaîne carbonée de 1 à 3 atomes de carbone, linéaire ou ramifiée, plus particulièrement un méthyle, éthyle, propyle, 1-méthyléthyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₅-cycloalkyle représente un groupe carboné cyclique de 3 à 5 atomes de carbone, plus particulièrement un cyclopropyle, cyclobutyle, cyclopentyle ;
- alcènylène, un groupe aliphatique insaturé divalent, plus particulièrement un éthylène ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon différentes méthodes, illustrées par les schémas qui suivent.

Ainsi une première méthode (schéma 1) de préparation des composés de formule générale (I) consiste à faire réagir une amine de formule générale (II), dans laquelle R₁, R₂, n et A sont tels que définis ci-dessus, avec un carbonate de formule générale (III), dans laquelle U représente un atome d'hydrogène ou un groupement nitro et R₃ est tel que défini ci-dessus, dans un solvant tel que le toluène ou le dichloroéthane, à une température comprise entre 0 et 80°C. 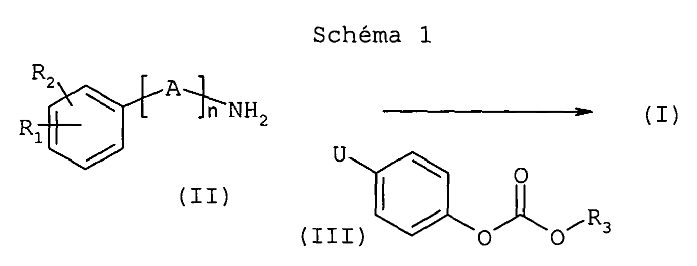 Selon une deuxième méthode, les composés de formule générale (I) pour lesquels R₃ représente plus particulièrement un phényle éventuellement substitué, peuvent être préparés en faisant réagir une amine de formule générale (II), telle que définie ci-dessus, avec un chloroformiate d'aryle de formule générale (IIIa) 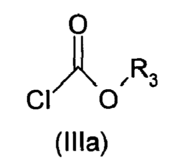 où R₃ représente un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy ,
dans un solvant tel que le dichlorométhane ou le dichloroéthane en présence d'une base telle que la triéthylamine ou la diisopropyléthylamine, à une température comprise entre 0°C et la température de reflux du solvant.

Les carbonates de formule générale (III) peuvent être préparés selon toute méthode décrite dans la littérature, par exemple par réaction d'un alcool de formule générale HOR₃ avec le chloroformiate de phényle ou de *para-*nitrophényle, en présence d'une base telle que la triéthylamine ou la diisopropyléthylamine.
Les composés de formules générales (II) et (IIIa) sont disponibles dans le commerce ou décrits dans la littérature, ou bien peuvent être préparés selon des méthodes qui y sont décrites ou qui sont connues de l'homme du métier.

Quand R₂ représente un groupe de type aryle ou hétéroaryle dans un composé de formule (I) ou (II), l'introduction de R₂ sur le cycle phényle peut être réalisée par réaction d'un dérivé d'un composé de formule générale (I) ou (II) dont le cycle phényle porte un atome de chlore , de brome, d'iode ou un groupement triflate dans la position où l'on souhaite introduire R₂, avec un dérivé d'acide boronique d'aryle ou d'hétéroaryle suivant les conditions de réaction de Suzuki (Chem. Rev. (1995), 95, 2457-2483 ; Angew. Chem. Int., Ed. (1999), 38, 3387-3388), ou avec un dérivé tri-alkylstanneux d'aryle ou d'hétéroaryle suivant les conditions de réaction de Stille (Angew. Chem. Int. Ed. Engl. (1986), 25, 508-524).

Les exemples suivant illustrent la préparation de quelques composés de l'invention. Ils ne sont pas limitatifs et ne font qu'illustrer l'invention. Les spectres R.M.N. et/ou la LC-MS (Liquid Chromatography coupled to Mass Spectroscopy) confirment les structures et les puretés des composés obtenus.
PF(°C) représente le point de fusion en degrés Celsius.
Les numéros indiqués entre parenthèses dans les titres des exemples correspondent à ceux de la 1ère colonne du tableau ci-après.
La nomenclature UICPA (Union Internationale de Chimie Pure et Appliquée - IUPAC en anglais) a été utilisée pour la dénomination des composés dans les exemples suivants. Par exemple, pour le groupe biphényle, la numérotation suivante a été respectée :

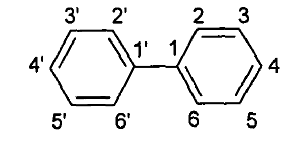

### Exemple 1 (Composé N°1) 1,1'-biphényl-4-ylméthylcarbamate de 2,2,2-trifluoroéthyle

A une solution de 0,3 g (1,49 mmole) de chloroformiate de *para*-nitrophényle dans 15 ml de chlorure de méthylène on ajoute, goutte à goutte et à température ambiante, 0,119 ml (1,64 mmole) de 2,2,2-trifluoroéthanol et 0,306 ml (1,49 mmole) de N,N-diisopropyléthylamine. On agite le mélange à température ambiante pendant 2 h, puis on ajoute 0,275 g (1,5 mmole) de 4-phénylbenzylamine. On ajoute ensuite, goutte à goutte et à température ambiante, de la N,N-diisopropyléthylamine jusqu'à disparition du précipité qui s'est formé. On agite la solution transparente ainsi obtenue à température ambiante pendant 1 h. On dilue le milieu réactionnel avec 10 ml de chlorure de méthylène supplémentaires et on lave avec une solution aqueuse saturée en chlorure d'ammonium et avec une solution aqueuse saturée de chlorure de sodium. On sépare les phases et on sèche la phase organique sur sulfate de sodium. On filtre, on concentre le filtrat sous pression réduite et on purifie le résidu par chromatographie sur gel de silice avec du chlorure de méthylène.
On obtient 0, 215 g de solide blanc.
LC-MS : 310
PF(°C) : 123-125°C
RMN^{1H} (DMSO-d₆) δ(ppm) :8,25 (t, 1H); 7,75 - 7,30 (m, 9H);
4,65 (q, 2H); 4,25 (d, 2H).

### Exemple 2 (Composé N°19) 2-(1,1'-biphényl-4-yl)éthylcarbamate de 2,2,2-trifluoroéthyle

On procède de manière analogue à l'exemple 1 en remplaçant la 4-phénylbenzylamine par la 2-(4-biphényl)éthylamine.
On obtient 0,311 g de solide blanc.
LC-MS : 324
PF(°C) : 79-81°C
RMN^{1H} (DMSO-d₆) δ(ppm) : 7,80 - 7,20 (m, 10H); 4,65 (q, 2H);
3,30 (m, 2H); 2,75 (t, 2H).

### Exemple 3 (Composé N°10) 4-phényloxybenzylcarbamate de 2,2,2-trifluoroéthyle

On procède de manière analogue à l'exemple 1 en remplaçant la 4-phénylbenzylamine par la 4-phényloxybenzylamine.
On obtient 0,252 g de solide blanc.
LC-MS : 326
PF(°C): 145-148°C
RMN^{1H} (DMSO-d₆) δ(ppm) : 8,15 (t, 1H); 7,40 - 6,90 (m, 9H);
4,65 (q, 2H); 4,20 (d, 2H).

### Exemple 4 (Composé N°3) 1,1'-biphényl-4-ylméthylcarbamate de 4-fluorophényle

A une solution de 0,107 g (0,58 mmole) de 4-phénylbenzyl amine dans 4 ml de chlorure de méthylène, on ajoute, goutte à goutte et à température ambiante, 0.069 ml (0,522 mmole) de chloroformiate de 4-fluorophényle et 0,149 ml (0,82 mmole) de *N,N*-diisopropyléthylamine. On agite le mélange à température ambiante pendant 1h. On dilue le milieu réactionnel avec 2 ml de chlorure de méthylène supplémentaires et on lave avec une solution aqueuse saturée en chlorure d'ammonium et avec une solution aqueuse saturée de chlorure de sodium. On sépare les phases et on filtre la phase organique à travers un fritté hydrophobe. On concentre le filtrat sous pression réduite et on lave le résidu solide avec 5 ml de diisopropyléther.
On obtient 0,136 g de solide blanc.
LC-MS : 322
PF(°C) : 155-157°C
RMN^{1H} (DMSO-d₆) δ(ppm) : 8,30 (t, 1H); 7,70 - 7,10 (m, 13H);
4,30 (d, 2H).

### Exemple 5 (Composé N°26) 2-(1,1'-biphényl-4-yl)éthylcarbamate de 4-méthylphényle

On procède de manière analogue à l'exemple 4 en remplaçant la 4-phénylbenzylamine par la 2-(4-biphényl)éthylamine et le chloroformiate de 4-fluorophényle par le chloroformiate de 4-méthylphényle.
On obtient 0,126 g de solide blanc.
LC-MS : 332
PF(°C) : 172-174°C
RMN^{1H} (DMSO-d₆) δ(ppm) : 7,75 (t, 1H); 7,70 - 7,30 (m, 9H); 7,10 (d, 2H); 6,90 (d, 2H); 3,30 (m, 2H); 2,80 (t, 2H); 2,25 (s, 3H).

### Exemple 6 (Composé N°16) 4-phényloxybenzylcarbamate de 4-méthoxyphényle

On procède de manière analogue à l'exemple 4 en remplaçant la 4-phénylbenzylamine par la 4-phényloxybenzylamine et le chloroformiate de 4-fluorophényle par le chloroformiate de 4-méthoxyphényle.
On obtient 0,137 g de solide blanc.
LC-MS : 350
PF(°C) :89-91°C
RMN^{1H} (DMSO-d₆) δ(ppm) : 8,20 (t, 1H); 7,45 - 7,25 (m, 4H);
7,20 - 6,80 (m, 9H); 4,25 (d, 2H); 3,75 (s, 3H).

Le tableau qui suit illustre les structures chimiques et les propriétés physiques de quelques composés selon l'invention. Dans ce tableau « n.d. » signifie que le point de fusion n'a pas pu être déterminé (produit sous forme de gomme par exemple) .

**Tableau**

| | | | | | |
|---|---|---|---|---|---|
| 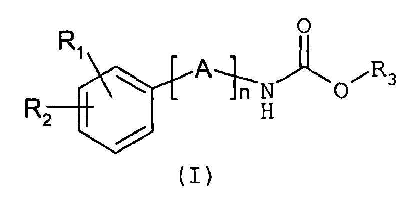 | | | | | |

| **N°** | **[A]ₙ** | **R₁** | **R₂** | **R₃** | **PF(°C)** |
|---|---|---|---|---|---|
| 1. | CH₂ | H | 4-phényl | CH₂CF₃ | 123-125 |
| 2. | CH₂ | H | 4-phényl | phényl | 158-162 |
| 3. | CH₂ | H | 4-phényl | 4-F-phényl | 155-157 |
| 4. | CH₂ | H | 4-phényl | 2-Cl-phényl | 128-130 |
| 5. | CH₂ | H | 4-phényl | 4-Cl-phényl | 161-164 |
| 6. | CH₂ | H | 4-phényl | 2-CH₃O-phényl | 186-188 |
| 7. | CH₂ | H | 4-phényl | 4-CH₃O-phényl | 153-156 |
| 8. | CH₂ | H | 4-phényl | 4-CH₃-phényl | 153-155 |
| 9. | CH₂ | H | 4-phényl | 3-CF₃-phényl | n.d. |
| 10. | CH₂ | H | 4-phényloxy | CH₂CF₃ | 145-148 |
| 11. | CH₂ | H | 4-phényloxy | phényl | 99-101 |
| 12. | CH₂ | H | 4-phényloxy | 4-F-phényl | 82-84 |
| 13. | CH₂ | H | 4-phényloxy | 2-Cl-phényl | 106-109 |
| 14. | CH₂ | H | 4-phényloxy | 4-Cl-phényl | 90-93 |
| 15. | CH₂ | H | 4-phényloxy | 2-CH₃O-phényl | 84-86 |
| 16. | CH₂ | H | 4-phényloxy | 4-CH₃O-phényl | 89-91 |
| 17. | CH₂ | H | 4-phényloxy | 4-CH₃-phényl | 105-107 |
| 18. | CH₂ | H | 4-phényloxy | 3-CF₃-phényl | n.d. |
| 19. | CH₂CH₂ | H | 4-phényl | CH₂CF₃ | 79-81 |
| 20. | CH₂CH₂ | H | 4-phényl | phényl | 150-152 |
| 21. | CH₂CH₂ | H | 4-phényl | 4-F-phényl | 160-163 |
| 22. | CH₂CH₂ | H | 4-phényl | 2-Cl-phényl | 131-133 |
| 23. | CH₂CH₂ | H | 4-phényl | 4-Cl-phényl | 167-169 |
| 24. | CH₂CH₂ | H | 4-phényl | 2-CH₃O-phényl | 116-119 |
| 25. | CH₂CH₂ | H | 4-phényl | 4-CH₃O-phényl | 158-160 |
| 26. | CH₂CH₂ | H | 4-phényl | 4-CH₃-phényl | 172-174 |
| 27. | CH₂CH₂ | H | 4-phényl | 3-CF₃-phényl | 132-135 |
| 28. | CH₂CH₂CH₂ | H | H | phényl | 62-65 |
| 29. | CH₂CH₂CH₂ | H | H | 4-F-phényl | 61-63 |
| 30. | CH₂CH₂CH₂ | H | H | 4-Cl-phényl | 53-56 |
| 31. | CH₂CH₂CH₂ | H | H | 4-CH₃-phényl | 79-81 |
| 32. | CH₂CH₂CH₂CH₂ | H | H | phényl | 76-78 |
| 33. | CH₂CH₂CH₂CH₂ | H | H | 4-F-phényl | 91-93 |
| 34. | CH₂CH₂CH₂CH₂ | H | H | 4-Cl-phényl | 95-97 |
| 35. | CH₂CH₂CH₂CH₂ | H | H | 4-CH₃-phényl | 91-93 |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005) et (Journal of Pharmacology and Experimented Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide.
Dans ces conditions, les composés les plus actifs de l'invention présentent des CI₅₀ (concentration inhibant de 50% l'activité enzymatique contrôle de la FAAH) comprises entre 0,001 et 1 µM.

Il apparaît donc que les composés selon l'invention ont une activité inhibitrice sur l'enzyme FAAH.

L'enzyme FAAH (Chemistry and Physics of Lipids, (2000), 108, 107-121) catalyse l'hydrolyse des dérivés endogènes d'amides et d'esters de différents acides gras tels que la N-arachidonoyléthanolamine (anandamide), la N-palmitoyl-éthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ ou tous autres substrats métabolisés par l'enzyme FAAH, sont impliqués.
On peut par exemple citer les maladies et les affections suivantes :
la douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ; les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires ; l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires , virales ou bactériennes : SIDA, méningites ;
les maladies inflammatoires, notamment les maladies articulaires : arthrite, arthrite rhumatoïde, ostéoarthrite, spondylite, goutte, vascularite, maladie de Crohn, syndrome du colon irritable ;
l'ostéoporose ;
les affections oculaires : hypertension oculaire, glaucome ; les affections pulmonaires : maladies des voies respiratoires, bronchospasmes, toux, asthme, bronchite chronique, obstruction chronique des voies respiratoires, emphysème ;
les maladies gastro-intestinales: syndrome du colon irritable, désordres inflammatoires intestinaux, ulcères, diarrhées ;
l'incontinence urinaire et l'inflammation vésicale.

L'utilisation des composés selon l'invention pour la préparation d'un médicament destiné à traiter les pathologies ci-dessus mentionnées fait partie intégrante de l'invention.

L'utilisation des composés suivants pour la préparation d'un médicament destiné à traiter les pathologies ci-dessus mentionnées fait également partie intégrante de l'invention :
- benzylcarbamate de 2,2,2-trifluoroéthyle;
- 4-méthoxybenzylcarbamate de 2,2,2-trifluoroéthyle ;
- 2-[4-(phénylméthoxy)phényl]éthylcarbamate de 4-nitrophényle;
- 2-(4-chlorophényl)éthylcarbamate de 4-chloro-2-nitrophényle;
- 2-(3,4-diméthoxyphényl)éthylcarbamate de 4-nitrophényle;
- 2-(3,4-diméthoxyphényl)éthylcarbamate de phényle;
- 2-(4-méthylphényl)éthylcarbamate de 4-cyanophényle;
- 2-(4-chlorophényl)éthylcarbamate de 2,4,5-trichlorophényle;
- 4-chlorobenzylcarbamate de phényle;
- 4-méthoxybenzylcarbamate de phényle;
- 2-(4-méthoxyphényl)éthylcarbamate de 4-fluorophényle;
- 3-nitrobenzylcarbamate de phényle;
- 4-méthoxybenzylcarbamate de 4-cyanophényle;
- 3-chlorobenzylcarbamate de phényle;
- 3,4-dichlorobenzylcarbamate de phényle;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(2-amino-4-thiazolyl)phényl]éthylcarbamate de phényle ;
- 4-bromobenzylcarbamate de 2,3,4,5,6-pentafluorophényle.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

L'invention a également pour objet des médicaments qui comprennent un composé choisi parmi la liste des composés suivants, ou un sel, ou encore un hydrate ou un solvat pharmaceutiquement acceptable de ce composé :
- benzylcarbamate de 2,2,2-trifluoroéthyle;
- 4-méthoxybenzylcarbamate de 2,2,2-trifluoroéthyle ;
- 2-[4-(phénylméthoxy)phényl]éthylcarbamate de 4-nitrophényle;
- 2-(4-chlorophényl)éthylcarbamate de 4-chloro-2-nitrophényle;
- 2-(3,4-diméthoxyphényl)éthylcarbamate de 4-nitrophényle;
- 2-(3,4-diméthoxyphényl)éthylcarbamate de phényle;
- 2-(4-méthylphényl)éthylcarbamate de 4-cyanophényle;
- 2-(4-chlorophényl)éthylcarbamate de 2,4,5-trichlorophényle;
- 4-chlorobenzylcarbamate de phényle;
- 4-méthoxybenzylcarbamate de phényle;
- 2-(4-méthoxyphényl)éthylcarbamate de 4-fluorophényle;
- 3-nitrobenzylcarbamate de phényle;
- 4-méthoxybenzylcarbamate de 4-cyanophényle;
- 3-chlorobenzylcarbamate de phényle;
- 3,4-dichlorobenzylcarbamate de phényle;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(2-amino-4-thiazolyl)phényl]éthylcarbamate de phényle ;
- 4-bromobenzylcarbamate de 2,3,4,5,6-pentafluorophényle.

Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.

Selon un autre de ses aspects, la présente invention concerne des compositions pharmaceutiques renfermant en tant que principe actif, au moins un composé choisi parmi la liste des composés suivants. Ces compositions pharmaceutiques contiennent une dose efficace d'un composé choisi parmi la liste des composés suivants, ou un sel, ou un hydrate, ou un solvat pharmaceutiquement acceptable dudit composé, et éventuellement un ou plusieurs excipients pharmaceutiquement acceptables :
- benzylcarbamate de 2,2,2-trifluoroéthyle;
- 4-méthoxybenzylcarbamate de 2,2,2-trifluoroéthyle ;
- 2-[4-(phénylméthoxy)phényl]éthylcarbamate de 4-nitrophényle;
- 2-(4-chlorophényl)éthylcarbamate de 4-chloro-2-nitrophényle;
- 2-(3,4-diméthoxyphényl)éthylcarbamate de 4-nitrophényle;
- 2-(3,4-diméthoxyphényl)éthylcarbamate de phényle;
- 2-(4-méthylphényl)éthylcarbamate de 4-cyanophényle;
- 2-(4-chlorophényl)éthylcarbamate de 2,4,5-trichlorophényle;
- 4-chlorobenzylcarbamate de phényle;
- 4-méthoxybenzylcarbamate de phényle;
- 2-(4-méthoxyphényl)éthylcarbamate de 4-fluorophényle;
- 3-nitrobenzylcarbamate de phényle;
- 4-méthoxybenzylcarbamate de 4-cyanophényle;
- 3-chlorobenzylcarbamate de phényle;
- 3,4-dichlorobenzylcarbamate de phényle;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(2-amino-4-thiazolyl)phényl]éthylcarbamate de phényle ;
- 4-bromobenzylcarbamate de 2,3,4,5,6-pentafluorophényle.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus ou l'un des composés suivants :
- benzylcarbamate de 2,2,2-trifluoroéthyle;
- 4-méthoxybenzylcarbamate de 2,2,2-trifluoroéthyle ;
- 2-[4-(phénylméthoxy)phényl]éthylcarbamate de 4-nitrophényle;
- 2-(4-chlorophényl)éthylcarbamate de 4-chloro-2-nitrophényle;
- 2-(3,4-diméthoxyphényl)éthylcarbamate de 4-nitrophényle;
- 2-(3,4-diméthoxyphényl)éthylcarbamate de phényle;
- 2-(4-méthylphényl)éthylcarbamate de 4-cyanophényle;
- 2-(4-chlorophényl)éthylcarbamate de 2,4,5-trichlorophényle;
- 4-chlorobenzylcarbamate de phényle;
- 4-méthoxybenzylcarbamate de phényle;
- 2-(4-méthoxyphényl)éthylcarbamate de 4-fluorophényle;
- 3-nitrobenzylcarbamate de phényle;
- 4-méthoxybenzylcarbamate de 4-cyanophényle;
- 3-chlorobenzylcarbamate de phényle;
- 3,4-dichlorobenzylcarbamate de phényle;
- 2-(4-hydroxyphényl)éthylcarbamate de 4-nitrophényle ;
- 2-[4-(2-éthyl-5,7-diméthyl-3H-imidazo[4,5-b]pyridin-3-yl)phényl]éthylcarbamate de phényle ;
- 2-[4-(2-amino-4-thiazolyl)phényl]éthylcarbamate de phényle ;
- 4-bromobenzylcarbamate de 2,3,4,5,6-pentafluorophényle ;
ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.
Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, inraoculaire, intranasale, par inhalation, les formes d'administration sous-cutanée, intramusculaire ou intra-veineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également une méthode de traitement des pathologies ci-dessus indiquées qui comprend l'administration d'une dose efficace d'un composé selon l'invention, d'un de ces sels pharmaceutiquement acceptables, d'un solvat ou d'un hydrate dudit composé.

## Revendications

1. Composé de formule (I): 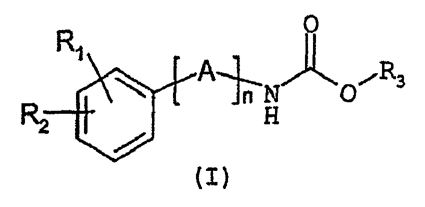 dans laquelle :
| **[A]ₙ** | **R₁** | **R₂** | **R₃** |
|---|---|---|---|
| CH₂ | H | 4-phényl | phényl |
| CH₂ | H | 4-phényl | 4-F-phényl |
| CH₂ | H | 4-phényl | 2-Cl-phényl |
| CH₂ | H | 4-phényl | 4-Cl-phényl |
| CH₂ | H | 4-phényl | 2-CH₃O-phényl |
| CH₂ | H | 4-phényl | 4-CH₃O-phényl |
| CH₂ | H | 4-phényl | 4-CH₃-phényl |
| CH₂ | H | 4-phényl | 3-CF₃-phényl |
| CH₂ | H | 4-phényloxy | phényl |
| CH₂ | H | 4-phényloxy | 4-F-phényl |
| CH₂ | H | 4-phényloxy | 2-Cl-phényl |
| CH₂ | H | 4-phényloxy | 4-Cl-phényl |
| CH₂ | H | 4-phényloxy | 2-CH₃O-phényl |
| CH₂ | H | 4-phényloxy | 4-CH₃O-phényl |
| CH₂ | H | 4-phényloxy | 4-CH₃-phényl |
| CH₂ | H | 4-phényloxy | 3-CF₃-phényl |
| CH₂CH₂ | H | 4-phényl | phényl |
| CH₂CH₂ | H | 4-phényl | 4-F-phényl |
| CH₂CH₂ | H | 4-phényl | 2-Cl-phényl |
| CH₂CH₂ | H | 4-phényl | 4-Cl-phényl |
| CH₂CH₂ | H | 4-phényl | 2-CH₃O-phényl |
| CH₂CH₂ | H | 4-phényl | 4-CH₃O-phényl |
| CH₂CH₂ | H | 4-phényl | 4-CH₃-phényl |
| CH₂CH₂ | H | 4-phényl | 3-CF₃-phényl |
| CH₂CH₂CH₂ | H | H | phényl |
| CH₂CH₂CH₂ | H | H | 4-F-phényl |
| CH₂CH₂CH₂ | H | H | 4-Cl-phényl |
| CH₂CH₂CH₂ | H | H | 4-CH₃-phényl |
| CH₂CH₂CH₂CH₂ | H | H | phényl |
| CH₂CH₂CH₂CH₂ | H | H | 4-F-phényl |
| CH₂CH₂CH₂CH₂ | H | H | 4-Cl-phényl |
| CH₂CH₂CH₂CH₂ | H | H | 4-CH₃-phényl |
à l'état de base, de sel d'addition à un acide, d'hydrate ou de solvat.

2. Procédé de préparation d'un composé de formule générale (I), 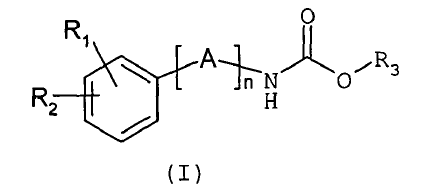 dans laquelle R₁, R₂, R₃, A et n sont tels que définis selon la revendication 1,
comprenant l'étape consistant à
faire réagir une amine de formule générale (II), 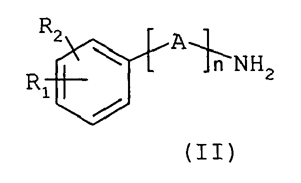 dans laquelle R₁, R₂, n et A sont tels que définis dans la formule générale (I), avec un carbonate de formule générale (III), 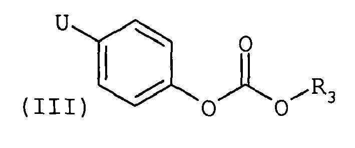 dans laquelle U représente un atome d'hydrogène ou un groupement nitro et R₃ est tel que défini dans la formule générale (I).

3. Procédé de préparation d'un composé de formule générale (I), 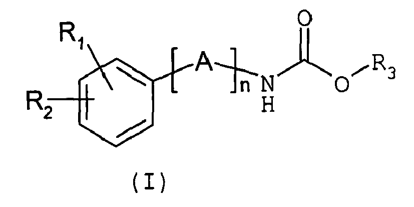 dans laquelle
R₁, R₂, A et n sont tels que définis selon la revendication 1 et
R₃ représente un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy, comprenant l'étape consistant à
faire réagir une amine de formule générale (II), 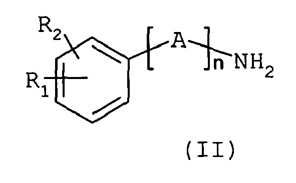 dans laquelle R₁, R₂, n et A sont tels que définis dans la formule générale (I), avec un chloroformiate d'aryle de formule générale (IIIa) 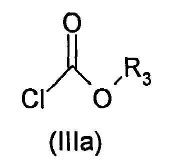 dans laquelle R₃ représente un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy.

4. Composition pharmaceutique contenant au moins un composé de formule (I) selon la revendication 1, à l'état de base, de sel, d'hydrate ou de solvat pharmaceutiquement acceptable et éventuellement un ou plusieurs excipients pharmaceutiquement acceptables.

5. Composé de formule (I) selon la revendication 1, à l'état de base, de sel, d'hydrate ou de solvat pharmaceutiquement acceptable, pour son utilisation comme médicament.

6. Utilisation d'un composé de formule (I) selon la revendication 1, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.
